(19)

**Europäisches Patentamt**
**European Patent Office**
**Office européen des brevets**

(11) **EP 4 578 905 A1**

(12) **EUROPEAN PATENT APPLICATION**
published in accordance with Art. 153(4) EPC

(43) Date of publication:
**02.07.2025 Bulletin 2025/27**

(21) Application number: **23856615.2**

(22) Date of filing: **22.08.2023**

(51) International Patent Classification (IPC):
**C08L 3/10** (2006.01)     **C08J 3/12** (2006.01)

(86) International application number:
**PCT/CN2023/114223**

(87) International publication number:
**WO 2024/041525 (29.02.2024 Gazette 2024/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.08.2022   CN 202211026120**

(71) Applicants:
• **Bloomage Biotechnology Corporation Limited**
  **Jinan, Shandong 250101 (CN)**
• **Bloomage Biotech (Tianjin) Co., Ltd.**
  **Tianjin 300270 (CN)**

(72) Inventors:
• **SUN, Shaojing**
  **Tianjin 300270 (CN)**
• **MA, Liang**
  **Tianjin 300270 (CN)**

• **JIN, Yan**
  **Jinan, Shandong 250101 (CN)**
• **CHEN, Qingping**
  **Jinan, Shandong 250101 (CN)**
• **LU, Zhen**
  **Jinan, Shandong 250101 (CN)**
• **WANG, Ruiyan**
  **Jinan, Shandong 250101 (CN)**
• **GUO, Xueping**
  **Jinan, Shandong 250101 (CN)**
• **AI, Fangmi**
  **Jinan, Shandong 250101 (CN)**
• **AI, Zheng**
  **Jinan, Shandong 250101 (CN)**
• **WANG, Enxu**
  **Tianjin 300270 (CN)**
• **ZHANG, Xianbao**
  **Tianjin 300270 (CN)**

(74) Representative: **Abel & Imray LLP**
  **Westpoint Building**
  **James Street West**
  **Bath BA1 2DA (GB)**

(54) **COMPOSITION, PREPARATION METHOD THEREFOR, AND USE THEREOF**

(57)     The present application provides a composition, comprising a wall material and γ-aminobutyric acid. The wall material is selected from one or two or more of β-cyclodextrin and derivatives thereof, maltodextrin, starch, chitosan and glucose. The composition of the present application can prevent the γ-aminobutyric acid from generating a Maillard reaction with a reducing sugar or carbonyl compound under the action of water, thereby not generating a brown or brown black macromolecular substance, and further not causing the color change of a product.

EP 4 578 905 A1

**Description**

TECHNICAL FIELD

The present application belongs to the technical field of raw material processing, and relates to a composition, preparation method and use thereof.

BACKGROUND ART

γ-aminobutyric acid (GABA) is a natural active amino acid that is widely found in animals, plants and microorganisms. It has received widespread attention due to its many physiological functions, including improving sleep quality, lowering blood pressure, fighting depression, fighting anxiety, delaying aging, and improving brain function. The food safety of GABA has been widely recognized by countries such as Japan and the United States. China also recognized GABA as a new food ingredient in 2009. Studies have shown that exogenous intake of a certain amount of GABA has physiological effects such as improving sleep quality and lowering blood pressure. Adding GABA, a functional factor, to a food and preparing it into a functional food may give full play to its natural efficacy.

GABA has strong hygroscopicity, so its powder has poor dispersibility and is not easy to mix evenly with other ingredients when used. Especially in the processing of food powders, due to the presence of free amino compounds in GABA, and they are easy to undergo the Maillard reaction with reducing sugars or carbonyl compounds to generate brown or even brown-black macromolecule substances, thereby causing the product to change color.

SUMMARY

In order to solve the problem in the prior art that γ-aminobutyric acid is prone to undergo Maillard reaction with reducing sugars or carbonyl compounds under the action of water, the present application provides a composition.

The technical solutions of this application are as follows:

1. A composition, comprising:

   a wall material and γ-aminobutyric acid;
   wherein the wall material is selected from one or more of β-cyclodextrin and derivatives thereof, maltodextrin, starch, chitosan and glucose.
   preferably, the DE value of the maltodextrin is 5-10, and the starch is oxidized starch.

2. The composition according to item 1, wherein
   the composition consists of a wall material and γ-aminobutyric acid.

3. The composition according to item 1, wherein
   the mass ratio of the wall material to the γ-aminobutyric acid is (1-4) : 1, preferably (2-4) : 1.

4. The composition according to item 1, wherein

   the wall material comprises β-cyclodextrin and derivatives thereof and maltodextrin;
   preferably, the mass ratio of the maltodextrin to the β-cyclodextrin and derivatives thereof is 1 : (0.01-0.5), preferably 1: (0.2-0.3).

5. The composition according to item 1, wherein

   the wall material comprises β-cyclodextrin and derivatives thereof and starch;
   preferably, the mass ratio of the starch to the β-cyclodextrin and derivatives thereof is 1 : (0.01-0.5), preferably 1 : (0.2-0.3).

6. A method for preparing the composition according to any one of items 1 to 5, wherein it comprises the steps of:

   preparing an aqueous solution A of the wall material;
   preparing an aqueous solution B of the γ-aminobutyric acid;
   mixing the aqueous solution of the γ-aminobutyric acid and the aqueous solution of the wall material to obtain an aqueous solution C, then stirring and spray-drying to obtain the composition.

7. The method according to item 6, wherein

in the aqueous solution C, the mass ratio of the sum of the mass of the wall material and the γ-aminobutyric acid to the aqueous solution C is (0.5-2.5) : 10, preferably (1-2) : 10.

8. A composite comprising the composition according to any one of items 1 to 5 or the composition prepared by the method according to any one of items 6 to 7.

9. Use of the composition according to any one of items 1 to 5 or the composition prepared by the method according to any one of items 6 to 7 in food or drug formulations.

10. Use of the composition according to any one of items 1 to 5 or the composition prepared by the method according to any one of items 6 to 7 in food and pharmaceutical raw materials.

11. Use of the composition according to any one of items 1 to 5 or the composition prepared by the method according to any one of items 6 to 7 in slowing down the Maillard reaction.

Compared with the prior art, the beneficial effects of this application are as follows:

1. The composition of the present application can prevent γ-aminobutyric acid from undergoing a Maillard reaction with reducing sugars or carbonyl compounds under the action of water, without generating brown or brown-black macromolecular substances or causing the product to change color.

2. The wall material in the composition of the present application can effectively block moisture from participating in the browning reaction and effectively protect the γ-aminobutyric acid.

3. The wall material in the composition of the present application has strong water absorption and can absorb water competitively to slow down the participation of moisture in the Maillard reaction of γ-aminobutyric acid in the formula food, thereby achieving a more significant effect.

4. The application is simple to operate, the production conditions are easy to control, and it is conducive to industrial production.

BRIEF DESCRIPTION OF THE DRAWINGS

FIG. 1 is the content stability results of some Examples;

FIG. 2 shows the color changes of the Maillard reaction in some Examples.

DETAILED DESCRIPTION

γ-aminobutyric acid is a white or slightly yellow crystalline powder with strong hygroscopicity, which is easily soluble in water, slightly soluble in hot ethanol, insoluble in organic solvents such as benzene, cold ethanol and ether. Moreover, γ-aminobutyric acid has a slight odor and is optically inactive, which is a polar substance with a melting point of 203-205°C, and has a relative molecular mass of 103.12, with a molecular formula of CHNO. The dissociation constants of γ-aminobutyric acid are: pK = 4.03, pK = 10.56. γ-Aminobutyric acid usually exists in the form of zwitterions in aqueous solution. Its dissociation mainly depends on the pH value, with an isoelectric point of 7.295. γ-aminobutyric acid is a non-protein amino acid with certain special chemical properties of amino acids, which can undergo alkylation reaction, acylation reaction, ninhydrin color development reaction, etc.

As a new food functional factor, γ-aminobutyric acid has attracted more and more attention. Studies have found that γ-aminobutyric acid can enhance the permeability of nerve cell membranes to Na, hyperpolarize the cell membrane, and increase the threshold for the initiation of action potentials. Therefore, γ-aminobutyric acid is an important inhibitory neurotransmitter in the central nervous system of humans and mammals. It can participate in a variety of metabolic activities in organisms and has strong physiological activity. Its production and application are becoming a hot research direction in the fields of food, medicine, animal feed, and agriculture. The physiological functions of γ-aminobutyric acid are mainly as follows:

A. Regulation of Cardiovascular Function

As an important inhibitory neurotransmitter in the central nervous system, γ-aminobutyric acid has a good regulatory effect on cardiovascular activity, mainly by regulating heart rate, lowering blood pressure and blood sugar. γ-aminobutyric acid has a certain pharmacological effect in lowering blood pressure, and the reason may be that there are specific nerve receptors in the cerebral blood vessels that can bind to γ-aminobutyric acid. When such nerve receptors bind to GABA, the purpose of lowering blood pressure can be achieved. Through experiments, Zhifang Mao et al. found that after long-term drinking of green tea containing γ-aminobutyric acid, the blood pressure of spontaneously hypertensive rats could be significantly reduced. Both Kazami and Inoue K found that if patients with hypertension continuously consume foods containing γ-aminobutyric acid, their blood pressure will be significantly reduced; but for people with normal blood pressure, their blood pressure will not change after continuous consumption, nor will it affect other indicators of the body, and there will be no side effects. γ-aminobutyric acid can also regulate the vasomotor center by blocking the sympathetic nerves, and cause blood vessels to dilate, thereby achieving the purpose of lowering blood pressure. γ-aminobutyric acid can lower blood pressure by inhibiting the activity of angiotensin-converting enzyme and antidiuretic hormone vasopressin. γ-aminobutyric acid can also inhibit the activity of glutamate decarboxylase, and effectively preven the decarboxylation of glutamate, thereby reducing blood ammonia levels and ensuring human health. Meanwhile, γ-aminobutyric acid will also promote the combination of glutamate and ammonia in the blood to form urea, thereby effectively eliminating ammonia poisoning in the body and enhancing liver and kidney function.

B. Treatment of Epilepsy

It was reported that the number of epilepsy patients in China has reached about 9 million. The causes of epilepsy are diverse and the mechanisms are complex, which are closely related to central neurotransmitters. At present, epilepsy has become the second most common disease in China, seriously affecting people's work and life. As an important inhibitory neurotransmitter in the human body, γ-aminobutyric acid has an improving effect on the treatment of epilepsy, insomnia, Parkinson's syndrome and other diseases. There are two types of amino acids in the central nervous system, namely excitatory amino acids and inhibitory amino acids. If the content of excitatory amino acids is too high, the nerve inhibitory effect will decrease, which may cause epilepsy. Many studies have found that when γ-aminobutyric acid is deficient, it will cause overexcitation of neurons. Furthermore, there is a specific binding site in the central nervous system, and binding to this site can increase the permeability of the nerve membrane to Cl, making the cell membrane potential at the resting potential level, thereby weakening the synaptic response to excitatory input. Therefore, γ-aminobutyric acid can increase the human body's anticonvulsant threshold, and can effectively treat refractory epilepsy. Okada et al. found that oral intake of rice germ foods containing γ-aminobutyric acid can promote sleep, calm nerves, resist anxiety, etc., and help improve symptoms of early mental disorders in the elderly and menopausal syndrome in women. In addition, the study found that the γ-aminobutyric acid content in cerebrospinal fluid was negatively correlated with the severity of epilepsy patients, and the γ-aminobutyric acid content was low. Therefore, epilepsy can be treated by directly increasing the content of γ-aminobutyric acid in cerebrospinal fluid. Studies have found that when the brain is severely ischemic, γ-aminobutyric acid can also reduce the neural excitation caused by glutamate, thereby reducing damage to nerve cells. In addition, γ-aminobutyric acid is also effective in treating spasticity and Parkinson's disease.

C. Promotion of Hormone Secretion

γ-aminobutyric acid regulates the secretion of multiple hormones in the endocrine system, including growth hormone, growth hormone mediators, thyroid hormones and sex hormones, and can effectively improve the body's metabolic level. γ-aminobutyric acid can directly stimulate the body to secrete the required hormones, which is more safer than directly taking exogenous hormones. Studies have shown that γ-aminobutyric acid can regulate the secretion of gonadotropin by inhibiting the excitation of certain nerves in the hypothalamus.

D. Improvement of Liver and Kidney Function

In addition to inhibiting the decarboxylation of glutamate to lower blood ammonia, γ-aminobutyric acid also has a diuretic effect, which allows the body to excrete excess salt and effectively relieve liver and kidney pressure. Studies have shown that γ-aminobutyric acid can activate kidney function. Moreover, γ-aminobutyric acid can also inhibit the activity of alkaline phosphatase, which is an important indicator for evaluating the activation of liver function.

E. Other functions

In addition, γ-aminobutyric acid also has physiological functions such as preventing obesity, promoting lipid metabolism, preventing vascular sclerosis, repairing skin, and delaying aging. Clinically, γ-aminobutyric acid can also treat diseases such as uremia and carbon monoxide poisoning. In addition, γ-aminobutyric acid also has insecticidal and deodorizing effects.

Due to the presence of free amino groups in γ-aminobutyric acid, when used as a food functional factor, it is easy to undergo the Maillard reaction with reducing sugars or carbonyl compounds under the action of water to produce brown or even brown-black macromolecule substances, thereby causing the product to change color.

The present application provides a composition comprising a wall material and γ-aminobutyric acid, wherein the wall material is selected from one or more of β-cyclodextrin and derivatives thereof, maltodextrin, starch, chitosan, and glucose.

In some embodiments of the present application, the maltodextrin is a maltodextrin with a low DE value, preferably the DE value of the maltodextrin is 5-10;

For example, the DE value of maltodextrin may be 5, 6, 7, 8, 9, 10, or any range therebetween.

In the present application, the DE value refers to the percentage of reducing sugars (calculated as glucose) in the dry matter of syrup. According to national standards, the higher the DE value, the higher the grade of glucose syrup. The DE value can be determined by any method known to those skilled in the art. The specifications of maltodextrin in light industry standards of China are divided into three categories, namely DE values ≤ 10, DE values ≤ 15, and DE values ≤ 20, respectively. Maltodextrin with low DE value is a widely used food ingredient, which has low sweetness, low hygroscopicity and good solubility, and often used as a carrier of flavor substances, humectant, film-forming agent and fat substitute.

In some embodiments of the present application, the DE value is determined by iodine titration.

In some embodiments of the present application, the starch is amylose and amylopectin and related modified starches, preferably oxidized starch.

Native starch is a carbohydrate found in nature in all plants, especially in plant seeds, roots and tubers, in which it serves as a nutrient reserve for the new growing season. Starch is a glucose polymer, in which anhydroglucose units are linked to each other by an α-D-glycosidic bond. The glucose chains are either straight or slightly branched amylose, or highly branched amylopectin. Typically, amylose molecules have a degree of polymerization of 1000 to 5000, while the amylopectin molecules have an average degree of polymerization of 1,000,000 or more. Starch can be isolated from, among other things, rice, corn, potato, wheat, *Manihot esculenta* (Manihot), cassava, barley, oats, millet, and sorghum. Modified starch is a starch prepared by at least partially degrading natural starch, for example by treating natural starch with inorganic acids, alkaline compounds, bleaching agents, oxidizing agents, enzymes, or acetyling agents. Oxidized starch is a modified starch obtained by oxidizing starch in an acid, alkali or neutral medium with an oxidant. Oxidized starch reduces the gelatinization temperature of starch, reduces the hot paste viscosity and increases the thermal stability; thus the product is white in color, transparent, and has good film-forming properties and good freeze-thaw resistance. As a low-viscosity and high-concentration thickener, it is widely used in the textile, papermaking, food and fine chemical industries.

In some embodiments of the present application, the oxidized starch is measured by any method known to those skilled in the art.

In some embodiments of the present application, the oxidized starch is determined by neutralization titration method.

Cyclodextrin (CD) is formed by the action of cyclodextrin glucose residue transferase on glucose polymers such as starch, glycogen, and maltooligosaccharide. The most common types are cyclodextrin α, β, and γ. Among the three types of cyclodextrins, β-cyclodextrin (β-CD) is a water-soluble, non-reducing white crystal or amorphous powder, the main structure of which is like a conical cylinder with a hole in the middle and open ends. In this hollow structure, the inner cavity is hydrophobic, and can contain many guest molecules, such as organic molecules, inorganic molecules, complexes and inert gas molecules. Therefore, they can form various inclusion compounds through weak interactions of non-covalent bonds, thereby changing the physical, chemical and biological properties of the guest molecules.

In some embodiments of the present application, the derivative of β-cyclodextrin is selected from one or more of carboxymethyl-β-cyclodextrin, glucosyl-β-cyclodextrin, ethylenediamine-β-cyclodextrin, hydroxyethyl-β-cyclodextrin, hydroxypropyl-β-cyclodextrin, sulfonate-β-cyclodextrin and quaternary ammonium-β-cyclodextrin.

In some embodiments of the present application, the composition consists of a wall material and γ-aminobutyric acid.

In some embodiments of the present application, the mass ratio of the wall material to the γ-aminobutyric acid is (1-4) : 1, preferably (2-4) : 1.

For example, the mass ratio of the wall material to the γ-aminobutyric acid is 1:1, 2:1, 3:1, 4:1 or, any range therebetween.

In some embodiments of the present application, the wall material comprises β-cyclodextrin and derivatives thereof and maltodextrin; preferably, the mass ratio of the maltodextrin to the β-cyclodextrin and derivatives thereof is 1 : (0.01-0.5), preferably 1: (0.2-0.3).

For example, the mass ratio of the maltodextrin to the β-cyclodextrin and derivatives thereof is 1:0.5, 1:0.45, 1:0.4, 1:0.35, 1:0.3, 1:0.25, 1:0.2, 1:0.15, 1:0.1, 1:0.05, 1:0.01, or any range therebetween.

In some embodiments of the present application, the wall material consists of β-cyclodextrin and derivatives thereof and maltodextrin.

In some embodiments of the present application, the wall material consists of β-cyclodextrin and maltodextrin.

In some embodiments of the present application, the wall material consists of β-cyclodextrin and maltodextrin with a DE value of 5-10.

In some embodiments of the present application, the wall material comprises β-cyclodextrin and derivatives thereof and starch; preferably, the mass ratio of the starch to the β-cyclodextrin and derivatives thereof is 1 : (0.01-0.5), preferably 1 : (0.2-0.3); for example, the mass ratio of the starch to the β-cyclodextrin and derivatives thereof is 1:0.5, 1:0.45, 1:0.4, 1:0.35, 1:0.3, 1:0.25, 1:0.2, 1:0.15, 1:0.1, 1:0.05, 1:0.01, or any range therebetween.

In some embodiments of the present application, the wall material consists of β-cyclodextrin and derivatives thereof and starch.

In some embodiments of the present application, the wall material consists of β-cyclodextrin and starch.

In some embodiments of the present application, the wall material consists of β-cyclodextrin and oxidized starch.

The present application provides a method for preparing the above-mentioned composition, wherein it comprises the steps of:

preparing an aqueous solution A of the wall material;

preparing an aqueous solution B of the γ-aminobutyric acid;

mixing the aqueous solution of the γ-aminobutyric acid and the aqueous solution of the wall material to obtain an aqueous solution C, then stirring and spray-drying to obtain the composition.

In some embodiments of the present application, in the aqueous solution C, the mass ratio of the sum of the mass of the wall material and the γ-aminobutyric acid to the aqueous solution C is (0.5-2.5) : 10, preferably (1-2) : 10.

For example, the sum of the mass of the wall material and the γ-aminobutyric acid and the mass of the aqueous solution C is 0.5:10, 0.75:10, 1:10, 1.25:10, 1.5:10, 1.75:10, 2:10, 2.25:10, 2.5:10, or any range therebetween.

In some embodiments of the present application, in the aqueous solution C, the mass ratio of the sum of the mass of the wall material and the γ-aminobutyric acid to the aqueous solution C also refers to the solid content, that is, in the aqueous solution C, the solid content is 5-25%, preferably 10-20%.

For example, in aqueous solution C, the solid content is 5%, 6%, 7%, 8%, 9%, 10%, 11%, 12%, 13%, 14%, 15%, 16%, 17%, 18%, 19%, 20%, 21%, 22%, 23%, 24%, 25%, or any range therebetween.

In some embodiments of the present application, when the C solution is stirred, the stirring temperature is 60-70°C, preferably 60-65°C.

For example, the stirring temperature may be 60°C, 61°C, 62°C, 63°C, 64°C, 65°C, 66°C, 67°C, 68°C, 69°C, 70°C, or any range therebetween.

In some embodiments of the present application, the inlet air temperature of the spray drying is 140-145°C, and the outlet air temperature of the spray drying is 95-105°C.

For example, the inlet air temperature for spray drying may be 140°C, 141°C, 142°C, 143°C, 144°C, 145°C, or any range therebetween.

For example, the outlet air temperature of the spray drying may be 95°C, 96°C, 97°C, 98°C, 99°C, 100°C, 101°C, 102°C, 103°C, 104°C, 105°C, or any range therebetween.

In some embodiments of the present application, an aqueous solution A of a wall material is prepared, wherein the wall material comprises β-cyclodextrin and derivatives thereof and maltodextrin; preferably, the mass ratio of the maltodextrin to the β-cyclodextrin and derivatives thereof is 1 : (0.01-0.5). Alternatively, the wall material comprises β-cyclodextrin and derivatives thereof and starch; preferably, the mass ratio of the starch to the β-cyclodextrin and derivatives thereof is 1 : (0.01-0.5), and the wall material solution is dissolved in deionized water at 70-80°C, then stirring for 10-15 minutes and cooling to 60-65°C to obtain a uniform solution.

In addition, an aqueous solution B of γ-aminobutyric acid is prepared, and the aqueous solution of γ-aminobutyric acid is mixed with an aqueous solution of a wall material to obtain an aqueous solution C. When the solution C is stirred, the stirring temperature is 60-65°C., that is, the temperature before spray drying is controlled at 60-65°C. In the aqueous solution C, the mass ratio of the sum of the mass of the wall material and the γ-aminobutyric acid to the mass of the aqueous solution C is (1-2) : 10, thereby ensuring the spray drying yield to reach more than 80%.

The present application provides a composite comprising the above composition.

The present application also provides use of the above composition in food, health care products and pharmaceutical formulations.

The present application also provides use of the above composition in food, health care products, and pharmaceutical raw materials.

The present application also provides use of the above composition in slowing down the Maillard reaction.

In the composition provided in the present application, on the one hand, the wall material used can partially wrap the γ-aminobutyric acid, and on the other hand, the wall material used can competitively absorb water, thereby reducing the probability of contact between γ-aminobutyric acid and water, and effectively blocking moisture so as to slow down the participation of moisture in the Maillard reaction in the formula food.

The ingredients used in the present application are safe and reliable, and the method can effectively alleviate the browning problem caused by the Maillard reaction between γ-aminobutyric acid and reducing sugars in formula foods, thereby expanding the application of γ-aminobutyric acid in the food field. As a result, the product is pure in color and tasteless, and can be dissolved in water-soluble products. Meanwhile, the preparation process is simple, the raw materials are easy to obtain, the product can obtained with a high yield, thereby facilitating to promote and apply it.

Table 1:

|  | Specification | Manufacturer |
|---|---|---|
| Oxidized starch | Model: 3262 | Foshan China Agricultural Starch Co. LTD |
| β-cyclodextrin | Content: ≥ 98.0% | Hebei Baiyou Biotechnology Co., Ltd |
| Maltodextrin (DE=5-10) | DE value: 5-10 | Shandong Xiwang Sugar Industry Co., Ltd. |

(continued)

| | Specification | Manufacturer |
|---|---|---|
| γ-aminobutyric acid | Content: ≥ 98% | Bloomage Biotechnology Corporation Limited |
| Corn starch | Content: ≥ 99% | Yufeng Industrial Group |
| Maltodextrin | Content: ≥ 99% | Shandong Xiwang Sugar Industry Co., Ltd. |

EXAMPLES

**Example 1**

An aqueous solution A of the wall material was prepared. 90g of oxidized starch and 22.5g of β-cyclodextrin were dissolved in 900mL of deionized water at 75°C, then stirring for 10 minutes and cooling to 60°C to obtain an aqueous solution A of the wall material. The mass ratio of oxidized starch to β-cyclodextrin is 1:0.25, and the sum of the mass of oxidized starch and β-cyclodextrin is 112.5g.

An aqueous solution B of γ-aminobutyric acid was prepared. 37.5g of γ-aminobutyric acid was weighed and dissolved in 100 mL of deionized water to obtain an aqueous solution B of γ-aminobutyric acid, and the weight percentage of γ-aminobutyric acid in the aqueous solution B is 37.5%.

The aqueous solution B of γ-aminobutyric acid was slowly added to the aqueous solution A of the wall material in proportion to obtain a solution C. In solution C, the mass ratio of the wall material to the γ-aminobutyric acid is 3:1, the mass ratio of the sum of the mass of the wall material and the γ-aminobutyric acid to the aqueous solution C is 1.5:10, the solid content is 15%. Particularly, when solution B was added to solution A to form solution C, the mixture was stirred to homogenize it. After the addition was completed, stirring and homogenizing were continued for 30 minutes, the stirring temperature was controlled at 62°C. Then spray drying was carried out with a spray dryer, the inlet air temperature of the spray dryer is 140°C, and the outlet air temperature of the spray dryer is 100°C, finally 136.8g of powdered solid, i.e., γ-aminobutyric acid sample 1 (S1) were prepared.

The ratio of the mass of the finally prepared powdered solid to the mass of the composition (a wall material and γ-aminobutyric acid) was recorded as the yield. The yield of Example 1 is 91.2%.

**Example 2**

The only difference of Example 2 over Example 1 is that: when the aqueous solution A of the wall material was prepared, 60g of oxidized starch and 15g of β-cyclodextrin were dissolved in 900mL of deionized water at 75°C, the mass ratio of oxidized starch to β-cyclodextrin is 1:0.25, the sum of the mass of oxidized starch and β-cyclodextrin is 75g, the mass ratio of wall material to γ-aminobutyric acid is 2:1, the solid content is 11.25%, and the other conditions were the same.

**Example 3**

The only difference of Example 3 over Example 1 is that: when the aqueous solution A of the wall material was prepared, 120g of oxidized starch and 30g of β-cyclodextrin were dissolved in 900mL of deionized water at 75°C, the mass ratio of oxidized starch to β-cyclodextrin is 1:0.25, the sum of the mass of oxidized starch and β-cyclodextrin is 150g, the mass ratio of wall material to γ-aminobutyric acid is 4:1, the solid content is 18.75%, and the other conditions were the same.

**Example 4**

The only difference of Example 4 over Example 1 is that: when the aqueous solution A of the wall material was prepared, 90g of maltodextrin (the DE value of maltodextrin is 5-10) and 22.5g of β-cyclodextrin were dissolved in 900 mL of deionized water at 75°C, the mass ratio of maltodextrin with a DE value of 5-10 and β-cyclodextrin is 1:0.25, the sum of the mass of maltodextrin with a DE value of 5-10 and β-cyclodextrin is 112.5g, the mass ratio of the wall material to γ-aminobutyric acid is 3:1, the solid content is 15%, and the other conditions were the same.

**Example 5**

The only difference of Example 5 over Example 1 is that: when the aqueous solution A of wall material was prepared, 90g of oxidized starch and 22.5g of β-cyclodextrin were dissolved in 1200 mL of deionized water at 75°C; when the aqueous solution B of γ-aminobutyric acid was prepared, 37.5g of γ-aminobutyric acid was weighed and dissolved in 300mL of deionized water to obtain an aqueous solution B of γ-aminobutyric acid; the mass ratio of oxidized starch to β-cyclodextrin is 1:0.25, the sum of the mass of oxidized starch and β-cyclodextrin is 112.5g, the mass ratio of wall material to γ-aminobutyric acid is 3:1, and the solid content of the obtained aqueous solution C is 10%, and the other conditions were the same.

**Example 6**

The only difference of Example 6 over Example 1 is that: when the aqueous solution A of wall material was prepared, 90g of oxidized starch and 22.5g of β-cyclodextrin were dissolved in 650 mL of deionized water at 75°C; when the aqueous solution B of γ-aminobutyric acid was prepared, 37.5g of γ-aminobutyric acid was weighed and dissolved in 100 mL of deionized water to obtain an aqueous solution B of γ-aminobutyric acid; the mass ratio of oxidized starch to β-cyclodextrin is 1:0.25, the sum of the mass of oxidized starch and β-cyclodextrin is 112.5g, the mass ratio of wall material to γ-aminobutyric acid is 3:1, and the solid content of the obtained aqueous solution C is 20%, and the other conditions were the same.

**Example 7**

The only difference of Example 7 over Example 1 is that: when the aqueous solution A of wall material was prepared, 75g of oxidized starch and 37.5g of β-cyclodextrin were dissolved in 900 mL of deionized water at 75°C, the mass ratio of oxidized starch to β-cyclodextrin is 1:0.5, and the sum of the mass of oxidized starch and β-cyclodextrin is 112.5 g; in the aqueous solution C, the mass ratio of the wall material to γ-aminobutyric acid is 3:1, the solid content is 15%, and the other conditions were the same.

**Example 8**

The only difference of Example 8 over Example 1 is that: when the aqueous solution A of wall material was prepared, 111.3g of oxidized starch and 1.2g of β-cyclodextrin were dissolved in 900 mL of deionized water at 75°C, the mass ratio of oxidized starch to β-cyclodextrin is 1:0.011, and the sum of the mass of oxidized starch and β-cyclodextrin is 112.5g; in the aqueous solution C, the mass ratio of the wall material to γ-aminobutyric acid is 3:1, the solid content is 15%, and the other conditions were the same.

**Example 9**

The only difference of Example 9 over Example 1 is that: when the aqueous solution A of wall material was prepared, 12.5g of maltodextrin (the DE value of maltodextrin is 5-10) was dissolved in 900 mL of deionized water at 75°C, the solid content is 15%, and the other conditions were the same.

**Example 10**

The only difference of Example 10 over Example 1 is that: when the aqueous solution A of wall material was prepared, 112.5g of oxidized starch was dissolved in 900mL of deionized water at 75°C, and the solid content is 15%, and the other conditions were the same.

**Example 11**

The only difference of Example 11 over Example 1 is that: when the aqueous solution A of wall material was prepared, 112.5g of β-cyclodextrin was dissolved in 900mL of deionized water at 75°C, the solid content is 15%, and the other conditions were the same.

**Example 12**

The only difference of Example 12 over Example 1 is that: when the aqueous solution A of wall material was prepared, 112.5g of corn starch was dissolved in 900mL of deionized water at 75°C, and the solid content is 15%, and the other conditions were the same.

## Example 13

The only difference of Example 13 over Example 1 is that: when the aqueous solution A of wall material was prepared, 112.5g of maltodextrin was dissolved in 900mL of deionized water at 75°C, the solid content is 15%, and the other conditions were the same.

## Example 14

The only difference of Example 14 over Example 1 is that: when the aqueous solution A of wall material was prepared, 90g of oxidized starch and 22.5g of β-cyclodextrin were dissolved in 1675mL of deionized water at 75°C; when the aqueous solution B of γ-aminobutyric acid was prepared, 37.5g of γ-aminobutyric acid was weighed and dissolved in 200mL of deionized water to obtain an aqueous solution B of γ-aminobutyric acid; in the obtained aqueous solution C, the sum of the mass of oxidized starch and β-cyclodextrin is 112.5g, in the aqueous solution C, the mass ratio of wall material to γ-aminobutyric acid is 3:1, the solid content is 8%, and the other conditions were the same.

## Example 15

The only difference of Example 15 over Example 1 is that: when the aqueous solution A of wall material was prepared, 90g of oxidized starch and 22.5g of β-cyclodextrin were dissolved in 500mL of deionized water at 75°C; when the aqueous solution B of γ-aminobutyric acid was prepared, 37.5g of γ-aminobutyric acid was weighed and dissolved in 100mL of deionized water to obtain an aqueous solution B of γ-aminobutyric acid; in the obtained aqueous solution C, the sum of the mass of oxidized starch and β-cyclodextrin is 112.5g. In the aqueous solution C, the mass ratio of wall material to γ-aminobutyric acid is 3:1, the solid content is 25%, and the other conditions were the same.

## Example 16

The only difference of Example 16 over Example 1 is that: when the aqueous solution A of wall material was prepared, 90g of corn starch and 22.5g of β-cyclodextrin were dissolved in 900mL of deionized water at 75°C, the mass ratio of corn starch to β-cyclodextrin is 1:0.25, and the sum of the mass of corn starch and β-cyclodextrin is 112.5g; in the aqueous solution C, the mass ratio of wall material to γ-aminobutyric acid is 3:1, the solid content is 15%, and the other conditions were the same.

## Example 17

The only difference of Example 17 over Example 1 is that: when the aqueous solution A of wall material was prepared, 90g of maltodextrin and 22.5g of β-cyclodextrin were dissolved in 900mL of deionized water at 75°C, the mass ratio of maltodextrin to β-cyclodextrin is 1:0.25, and the sum of the mass of maltodextrin and β-cyclodextrin is 112.5g; in the aqueous solution C, the mass ratio of wall material to γ-aminobutyric acid is 3:1, the solid content is 15%, and the other conditions were the same.

The parameters of Examples 1-17 are shown in Table 2.

| | Serial Number | Wall material | | | | γ-aminobutyric acid | Mass ratio of wall material and γ-aminobutyric acid | Solid content in solution C |
| | | Oxidized starch or maltodextrin | β-cyclodextrin | Sum of the mass of oxidized starch (or maltodextrin) and β-cyclodextrin | Mass ratio of oxidized starch and β-cyclodextrin or the mass ratio of maltodextrin and β-cyclodextrin | | | |
| Example 1 | S1 | Oxidized starch 90g | 22.5g | 112.5g | 1:0.25 | 37.5 | 3:1 | 15% |
| Example 2 | S2 | Oxidized starch 60g | 15g | 75g | 1:0.25 | 37.5 | 2:1 | 11.25% |
| Example 3 | S3 | Oxidized starch 120g | 30g | 150g | 1:0.25 | 37.5 | 4:1 | 18.75% |
| Example 4 | S4 | Maltodextrin (DE=5-10) 90g | 22.5g | 112.5g | 1:0.25 | 37.5 | 3:1 | 15% |
| Example 5 | S5 | Oxidized starch 90g | 22.5g | 112.5g | 1:0.25 | 37.5 | 3:1 | 10% |
| Example 6 | S6 | Oxidized starch 90g | 22.5g | 112.5g | 1:0.25 | 37.5 | 3:1 | 20% |
| Example 7 | S7 | Oxidized starch 75g | 37.5g | 112.5g | 1:0.5 | 37.5 | 3:1 | 15% |
| Example 8 | S8 | Oxidized starch 111.3g | 1.2g | 112.5g | 1:0.011 | 37.5 | 3:1 | 15% |
| Example 9 | S9 | Maltodextrin (DE=5-10) 112.5g | --- | --- | --- | 37.5 | 3:1 | 15% |
| Example 10 | S10 | Oxidized starch 112.5g | --- | --- | --- | 37.5 | 3:1 | 15% |
| Example 11 | S11 | --- | 112.5g | --- | --- | 37.5 | 3:1 | 15% |
| Example 12 | S12 | Corn starch 112.5g | --- | --- | --- | 37.5 | 3:1 | 15% |
| Example 13 | S13 | Maltodextrin 112.5g | --- | --- | --- | 37.5 | 3:1 | 15% |

EP 4 578 905 A1

(continued)

| | | Wall material | | | | | Mass ratio of wall material and γ-aminobutyric acid | Solid content in solution C |
|---|---|---|---|---|---|---|---|---|
| | Serial Number | Oxidized starch or maltodextrin | β-cyclodextrin | Sum of the mass of oxidized starch (or maltodextrin) and β-cyclodextrin | Mass ratio of oxidized starch and β-cyclodextrin or the mass ratio of maltodextrin and β-cyclodextrin | γ-aminobutyric acid | | |
| Example 14 | S14 | Oxidized starch 90g | 22.5g | 112.5g | 1:0.25 | 37.5 | 3:1 | 8% |
| Example 15 | S15 | Oxidized starch 90g | 22.5g | 112.5g | 1:0.25 | 37.5 | 3:1 | 25% |
| Example 16 | S16 | Corn starch 90g | 22.5g | 112.5g | 1:0.25 | 37.5 | 3:1 | 15% |
| Example 17 | S17 | Maltodextrin 90g | 22.5g | 112.5g | 1:0.25 | 37.5 | 3:1 | 15% |

**Experimental Example 1: Sample Stability Study**

Each of the samples of Examples 1-4, Examples 7-13 and Examples 16-17 was respectively mixed with maltose, lactose, glucose, fructose and arabinose in a ratio of 1:1, to carry out a high temperature accelerated test at 60°C. The color change was observed after 14 days, and the GABA content was detected according to the following method.

1. Instrument: High performance liquid chromatograph equipped with UV detector, autosampler and data processing system; Chromatographic column: Hypersil ODS C18, 5μm, 4.6×250mm (chromatographic column with equal or better separation effect); Analytical balance: the accuracy is 0.1mg; Ultrasonic dissolver.

2. Reagents: methanol: chromatographic grade; acetonitrile: chromatographic grade; o-phthalaldehyde (OPA); crystalline sodium acetate; glacial acetic acid; γ-aminobutyric acid standard: purity ≥ 99.0%; boric acid; sodium hydroxide

3. Analysis Steps

Preparation of Standard Solution: 0.5 g of γ-aminobutyric acid standard was accurately weighed to dissolve in water and make up to 100mL, mixing well and taking 10mL to make up to 100mL, filtering with a 0.22μm filter membrane, and collecting the filtrate as the standard solution.

Preparation of Sample Solution: 0.5-2.0 g of sample was accurately weighed to dissolve in water and make up to 100mL, mixing well and taking 10mL to make up to 100mL, filtering with a 0.22μm filter membrane, and collecting the filtrate as the sample solution to be tested.

Preparation of 0.4mol/L Boric Acid Buffer: 2.47 g of boric acid was weighed, about 80 mL of water was added, and the pH was adjusted to 10.2 with sodium hydroxide, making up to 100 mL with water.

Preparation of Derivatization Reagent: 0.1 g of o-phthalaldehyde (OPA) was weighed to dissolve in 1mL of acetonitrile, and 130μL of mercaptoethanol was added, making up to 100 mL with 0.4mol/L boric acid buffer.

4. Chromatographic Analysis Conditions

Mobile phase A: 7.5 g of crystalline sodium acetate was weighed to dissolve in water and make up to 1000mL, and 5% acetic acid was added dropwise to adjust the pH to 7.20±0.02, filtering and setting aside. Phase B: the chromatographic acetonitrile was filtered, setting aside. When running the method, the mobile phase ratio is: 75% of phase A + 25% of phase B. Flow rate: 1.0mL/min. Detection wavelength: 338nm. Column temperature: 40°C.

5. Sample Measurement

Pre-column derivatization was carried out by using an automatic sampler to perform chromatographic analysis. The retention time and peak area of the chromatographic peak were recorded to obtain the area ratio of the peak area and the concentration (calculated by dry product) of the standard solution. The operation was repeated for 6 times. The relative standard deviation should be less than 3%.

The prepared samples were selected for measurement. Each sample was prepared in duplicate, and each of them was measured for twice. The retention time and peak area of the chromatographic peak were recorded, in which the retention time of the sample and that of the standard solution should be consistent. The corresponding concentration of γ-aminobutyric acid was calculated by the external standard method.

The content of γ-aminobutyric acid in a sample is calculated according to the following formula:

$$X_1 = \frac{Ai \times \frac{m_s(1-n_s)\ C}{V_s}}{As \times \frac{m\ (1-n)}{V}} \times 100\%$$

wherein:

$X_1$- the content of the γ-aminobutyric acid in the sample;
$A_i$- the peak area of the γ-aminobutyric acid in the sample;
$m_s$-the mass of the γ-aminobutyric acid standard, in grams (g);
$n_s$- loss on drying of the γ-aminobutyric acid standard;
C- the purity of the γ-aminobutyric acid standard;
$V_s$- the dilution volume of the γ-aminobutyric acid standard, in milliliters (mL);
$A_s$- the peak area of the γ-aminobutyric acid standard;
m- the mass of the sample weighed, in grams (g);

n- the loss on drying of the sample;
V the dilution volume of the sample, in milliliters (mL).

The calculation result shall be rounded to one significant decimal place.

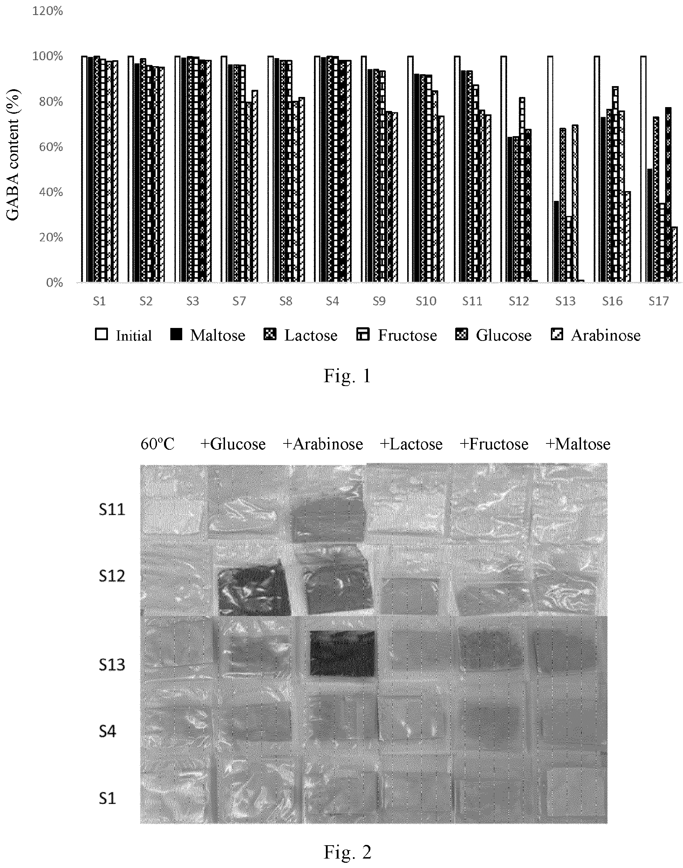

The results of Examples 1-4, Examples 7-13, and Examples 16-17 are shown in FIG. 1.

From the test of γ-aminobutyric acid content, it can be seen that when the wall materials are selected from maltodextrin (S9), oxidized starch (S10) and β-cyclodextrin (S11) with low DE values to compound with various sugars respectively, the content of γ-aminobutyric acid has a certain downward trend after being accelerated under high temperature conditions. Among them, the content of γ-aminobutyric acid is reduced to 60-70% after being compounded with glucose and arabinose under the condition that being accelerated under high temperature conditions. While the contents of the samples S12 and S13 prepared with corn starch and maltodextrin as wall materials are reduced to 67-80% and within 10% respectively after being compounded with glucose and arabinose under the condition that being accelerated under high temperature conditions, indicating that corn starch and maltodextrin have poor stability for γ-aminobutyric acid. Compared with Examples S7 and S8, samples S1-S4 are respectively compounded with glucose, fructose, lactose, arabinose and maltose. After being accelerated under high temperature conditions, the γ-aminobutyric acid content of S1-S3 and S4 is almost unchanged, and the effect is very good. The γ-aminobutyric acid contents in the samples of Examples S7 and S8 are reduced to about 80%; while the contents of samples S16 and S17, which are prepared by compounding corn starch and maltodextrin with β-cyclodextrin as wall materials respectively, are reduced to 75-80% and within 10% after being compounded with glucose and arabinose under the condition that being accelerated under high temperature conditions. The experimental results show that, the samples prepared by spray drying with oxidized starch and β-cyclodextrin as wall materials in a compounding ratio of 1:0.2 to 1:0.3 and γ-aminobutyric acid have better stability and can effectively slow down the Maillard reaction.

As shown in Figure 2, from the color reaction test, it can be seen that the samples prepared by spray drying of S1 (oxidized starch: β-cyclodextrin = 1:0.25), S4 (MD with low DE value: β-cyclodextrin = 1:0.25) together with S11 (β-cyclodextrin), S12 (corn starch), S13 (maltodextrin) and γ-aminobutyric acid have the smallest color change after being compounded with various sugars via being accelerated under high temperature conditions. From the color reaction test, it can be seen that the color change process is a process from white slowly changes to yellow or yellowish, and as the Maillard reaction time increases and the degree deepens, it gradually changes to brown or even black.

Table 3: Effect of solid content on yield

|     | Solid content | Yield  | Moisture |
| --- | ------------- | ------ | -------- |
| S1  | 15%           | 91.17% | 2.17%    |
| S5  | 10%           | 90.17% | 2.27%    |
| S6  | 20%           | 89.95% | 2.08%    |
| S14 | 8%            | 91.17% | 3.99%    |
| S15 | 25%           | 51.27% | 3.24%    |

It can be seen from the above Table 3 that the recovery rate (yield) of the sample decreases with the increase of the solid content. When the solid content is 8% (S14) and 25% (S15), and the moisture content is higher than 3%, the participation of moisture will aggravate the intensity of the Maillard reaction. In view of the comprehensive consideration of energy consumption, yield and quality, 15%-20% solid content is the optimal condition.

**Claims**

1. A composition, comprising:

   a wall material and γ-aminobutyric acid;
   wherein the wall material is selected from one or more of β-cyclodextrin and derivatives thereof, maltodextrin, starch, chitosan and glucose.
   preferably, the DE value of the maltodextrin is 5-10, and the starch is oxidized starch.

2. The composition according to claim 1, wherein
   the mass ratio of the wall material to the γ-aminobutyric acid is (1-4) : 1, preferably (2-4) : 1.

3. The composition according to claim 1, wherein

   the wall material comprises β-cyclodextrin and derivatives thereof and maltodextrin;
   preferably, the mass ratio of the maltodextrin to the β-cyclodextrin and derivatives thereof is 1 : (0.01-0.5), preferably 1: (0.2-0.3).

4. The composition according to claim 1, wherein

   the wall material comprises β-cyclodextrin and derivatives thereof and starch;
   preferably, the mass ratio of the starch to the β-cyclodextrin and derivatives thereof is 1 : (0.01-0.5), preferably 1 : (0.2-0.3).

5. A method for preparing the composition according to any one of claims 1 to 4, wherein it comprises the steps of:

   preparing an aqueous solution A of the wall material;
   preparing an aqueous solution B of the γ-aminobutyric acid;
   mixing the aqueous solution of the γ-aminobutyric acid and the aqueous solution of the wall material to obtain an aqueous solution C, then stirring and spray-drying to obtain the composition.

6. The method according to claim 5, wherein
   in the aqueous solution C, the mass ratio of the sum of the mass of the wall material and the γ-aminobutyric acid to the aqueous solution C is (0.5-2.5) : 10, preferably (1-2) : 10.

7. A composite comprising the composition according to any one of claims 1 to 4 or the composition prepared by the method according to any one of claims 5 to 6.

8. Use of the composition according to any one of claims 1 to 4 or the composition prepared by the method according to any one of claims 5 to 6 in food or drug formulations.

9. Use of the composition according to any one of claims 1 to 4 or the composition prepared by the method according to any one of claims 5 to 6 in food or pharmaceutical raw materials.

10. Use of the composition according to any one of claims 1 to 4 or the composition prepared by the method according to any one of claims 5 to 6 in slowing down the Maillard reaction.

Fig. 1

Fig. 2

## INTERNATIONAL SEARCH REPORT

| | International application No. |
|---|---|
| | **PCT/CN2023/114223** |

| **A.** | **CLASSIFICATION OF SUBJECT MATTER** |
|---|---|
| | C08L3/10(2006.01)i; C08J3/12(2006.01)i |

According to International Patent Classification (IPC) or to both national classification and IPC

| **B.** | **FIELDS SEARCHED** |
|---|---|

Minimum documentation searched (classification system followed by classification symbols)

IPC: C08J, C08L

Documentation searched other than minimum documentation to the extent that such documents are included in the fields searched

Electronic data base consulted during the international search (name of data base and, where practicable, search terms used)

DWPI; SIPOABS; CNABS; CNKI; Elsevier; Caplus: 壁材, 氨基丁酸, 环糊精, 麦芽糊精, 淀粉, 壳聚糖, 葡萄糖, wall, aminobutyric acid, cyclodextrin, maltodextrin, starch, chitosan, glucose

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 115322450 A (BLOOMAGE BIOTECHNOLOGY CORPORATION LIMITED et al.) 11 November 2022 (2022-11-11)<br>claims 1-10 | 1-10 |
| X | CN 104055788 A (HEILONGJIANG BAYI AGRICULTURAL UNIVERSITY) 24 September 2014 (2014-09-24)<br>claims 1-4 | 1, 2, 5-9 |
| A | CN 112568429 A (GUANGZHOU HANFANG PHARMACEUTICAL CO., LTD.) 30 March 2021 (2021-03-30)<br>entire document | 1-10 |
| A | JP 2008150350 A (UNITIKA LTD.) 03 July 2008 (2008-07-03)<br>entire document | 1-10 |
| A | US 2010196542 A1 (ROQUETTE FRERES) 05 August 2010 (2010-08-05)<br>entire document | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 December 2023** | **05 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/114223**

| Patent document cited in search report | | | Publication date (day/month/year) | Patent family member(s) | | | Publication date (day/month/year) |
|---|---|---|---|---|---|---|---|
| CN | 115322450 | A | 11 November 2022 | None | | | |
| CN | 104055788 | A | 24 September 2014 | None | | | |
| CN | 112568429 | A | 30 March 2021 | None | | | |
| JP | 2008150350 | A | 03 July 2008 | None | | | |
| US | 2010196542 | A1 | 05 August 2010 | KR | 20100059784 | A | 04 June 2010 |
| | | | | KR | 101553083 | B1 | 14 September 2015 |
| | | | | EP | 2173190 | A2 | 14 April 2010 |
| | | | | EP | 2173190 | B1 | 13 April 2016 |
| | | | | JP | 2010533485 | A | 28 October 2010 |
| | | | | JP | 5526025 | B2 | 18 June 2014 |
| | | | | DK | 2173190 | T3 | 01 August 2016 |
| | | | | TW | 200906314 | A | 16 February 2009 |
| | | | | TWI | 453042 | B | 21 September 2014 |
| | | | | CA | 2697601 | A1 | 26 February 2009 |
| | | | | CA | 2697601 | C | 23 August 2016 |
| | | | | WO | 2009024690 | A2 | 26 February 2009 |
| | | | | WO | 2009024690 | A3 | 29 October 2009 |
| | | | | US | 8883243 | B2 | 11 November 2014 |
| | | | | MX | 2010000718 | A | 31 March 2010 |
| | | | | FR | 2918845 | A1 | 23 January 2009 |
| | | | | FR | 2918845 | B1 | 30 November 2012 |

Form PCT/ISA/210 (patent family annex) (July 2022)